# FASCICULE DE BREVET EUROPEEN

(11) **EP 0 773 039 B1**
(45) Date de publication et mention de la délivrance du brevet: **20.08.2003**
(21) Numéro de dépôt: 96402379.0
(22) Date de dépôt: 08.11.1996
(51) Int. Cl.: A61N 1/39

(54) **Défibrillateur/cardioverteur implantable**
Implantierbarer Defibrillator/Kardiovertierer
Implantable defibrillator/cardioverter

(30) Priorité: 10.11.1995 FR 9513362
(43) Date de publication de la demande: 14.05.1997
(73) Titulaire: ELA MEDICAL (Société anonyme), 92541 Montrouge (FR)
(72) Inventeur: Ripart, Alain, 91190 Gif sur Yvette (FR)
(74) Mandataire: Dupuis-Latour, Dominique

(56) Documents cités:
- EP-A- 0 253 505
- EP-A- 0 522 693
- WO-A-95/16494
- US-A- 5 224 476
- US-A- 5 360 435
- US-A- 5 376 103

## Description

La présente invention concerne les dispositifs médicaux implantables actifs (au sens de la directive 90/385/CEE du 20 juin 1990 du Conseil des communautés européennes), et plus particulièrement la famille des appareils chargés de délivrer au coeur des impulsions électriques de haute énergie (c'est-à-dire dépassant notablement l'énergie fournie pour la simple stimulation) en vue de mettre fin à une tachyarythmie.

Ces dispositifs sont communément appelés "défibrillateurs implantables" ou "appareils de cardioversion" (étant entendu que l'invention couvre aussi bien les défibrillateurs/cardioverteurs/stimulateurs implantables que les défibrillateurs/stimulateurs implantables).

Ces dispositifs sont constitués de deux ensembles, à savoir un générateur d'impulsions et un système de sondes.

Le générateur d'impulsions est chargé de surveiller l'activité cardiaque et de générer des impulsions de haute énergie quand le coeur présente une arythmie ventriculaire susceptible d'être traitée. Quand cette énergie est comprise entre 0,1 et 10 J environ, on désigne cette thérapie sous le nom de "cardioversion" et le choc électrique est appelé "choc de cardioversion". Quand cette énergie est supérieure à 10 J environ, le choc électrique est alors appelé "choc de défibrillation".

À ce générateur sont connectées une ou plusieurs sondes munies d'électrodes dont le rôle est de distribuer au coeur cette énergie de façon appropriée.

On connaît plusieurs configurations différentes d'électrodes, décrites par exemple dans le US-A-5 376 103. L'une des configurations souvent employées consiste à utiliser deux électrodes, dont l'une est placée dans l'apex du ventricule droit et l'autre au voisinage de l'oreillette, par exemple dans le sinus coronaire, ou bien à l'intérieur même de l'oreillette. Par la suite, on désignera cette dernière électrode par le terme général d"'électrode auriculaire", qu'elle soit située dans l'oreillette ou au voisinage de celle-ci.

Il est en outre prévu au moins une électrode extérieure au coeur, qui peut notamment être le boîtier du générateur d'impulsions et/ou éventuellement une électrode sous-cutanée, appelée "patch", de grande surface et servant d'électrode(s) pour le recueil du courant injecté depuis l'intérieur du coeur.

En résumé, le système d'électrodes comporte, d'une part, une électrode auriculaire et une électrode ventriculaire et, d'autre part, une ou deux électrodes de grande surface extérieures au coeur.

Le choc de cardioversion ou de défibrillation peut être délivré selon plusieurs configurations. Ces diverses configurations, que l'on va décrire ci-dessous, sont choisies par des circuits de commutation internes au générateur d'impulsions ; on optimise ainsi l'efficacité du choc en choisissant la configuration d'électrodes la mieux appropriée à l'état du patient.

Les diverses configurations possibles sont les suivantes :
a) le choc est délivré entre l'électrode ventriculaire et l'électrode auriculaire ;
b) le choc est délivré entre l'électrode ventriculaire et l'une des électrodes extérieures au coeur (boîtier ou patch) ; dans une variante, ces deux électrodes extérieures sont en outre reliées électriquement entre elles au moment du choc de façon à augmenter le volume du muscle cardiaque traversé par le courant électrique ;
c) le choc est délivré entre, d'une part, l'électrode ventriculaire et, d'autre part, l'ensemble formé par l'électrode auriculaire et le boîtier et/ou le patch reliés ensemble, les liaisons électriques entre ces deux ou trois derniers éléments étant réalisées au moment de la délivrance du choc ;
d) le choc est délivré entre, d'une part, l'ensemble formé par l'électrode auriculaire et l'électrode ventriculaire reliées ensemble au moment de la délivrance du choc et, d'autre part, le boîtier et/ou le patch.

Le WO-A-95/16494 décrit en détail ces diverses configurations. La manière dont on peut opérer les différentes commutations à l'intérieur du boîtier pour y parvenir est par exemple décrite dans le US-A-5 376103 précité.

Par un choix approprié de la configuration, on peut ainsi optimiser le choc de cardioversion ou de défibrillation en fonction de la situation du patient.

Il a toutefois été observé (Florin et coll., *The Induction of Atrial Fibrillation with Low Energy Defibrillator Shock is related to Lead and Pulse Width*, publié dans le *Supplément to Circulation*, Vol. 92, n° 8, 15 octobre 1995, Abstracts from the 68th Scientific Sessions, Anaheim Convention Center, Anaheim, California, November 13-16, 1995, p. 143, abstract n° 0667) que, dans le cas des configurations décrites ci-dessus sous a), c) et d), qu'après la délivrance d'une impulsion de cardioversion, une proportion significative de patients développent une fibrillation auriculaire.

En d'autres termes, le choc de cardioversion réduit certes efficacement le trouble du rythme ventriculaire, mais en créant un risque de survenance d'une arythmie auriculaire.

En effet, dans les configurations connues a), c) et d) ci-dessus, l'électrode auriculaire est utilisée pour la délivrance du choc de cardioversion, et il circule donc un courant dont l'intensité permet d'arrêter la fibrillation ventriculaire, mais qui est suffisamment important pour risquer d'induire une fibrillation auriculaire.

L'un des buts de la présente invention est de minimiser, voire supprimer cet inconvénient.

Le dispositif de l'invention comprend une électrode auriculaire adaptée à être placée au voisinage ou à l'intérieur de l'oreillette, une électrode ventriculaire adaptée à être placée à l'intérieur du ventricule, au moins une électrode adaptée à être placée externe au coeur, et un boîtier générateur d'impulsions. Le boîtier comporte des moyens de délivrance d'une énergie de défibrillation ou de cardioversion et des moyens de commutation de configuration d'électrodes, qui déterminent entre quelles électrodes doit être appliquée l'énergie de défibrillation ou de cardioversion.

De façon caractéristique de l'invention, les moyens de commutation comprennent en outre des moyens pour, lorsqu'une énergie de cardioversion doit être délivrée, déconnecter l'électrode auriculaire des moyens de délivrance de l'énergie de défibrillation ou de cardioversion si bien que l'électrode auriculaire se trouve à un potentiel flottant, et, si la configuration d'électrodes déterminée par les moyens de commutation prévoit la commutation de l'électrode auriculaire, substituer à cette électrode l'une au moins des électrodes externes au coeur.

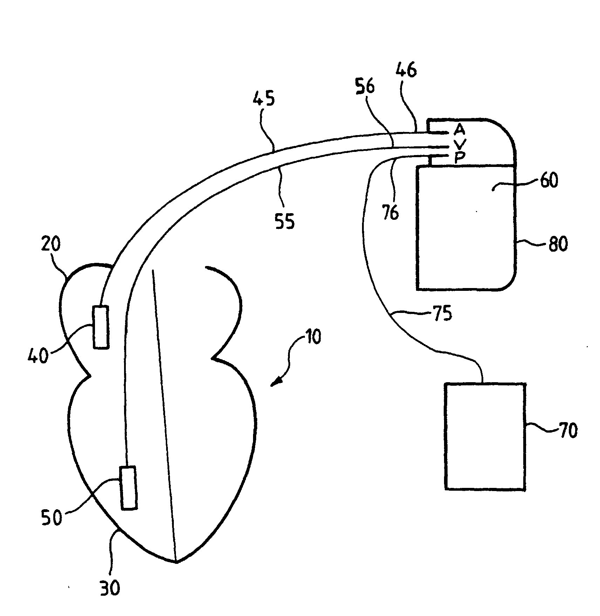

D'autres caractéristiques et avantages de l'invention apparaîtront à la lecture de la description ci-dessous, faite en référence à la figure unique annexée qui représente un dispositif selon l'invention (générateur et système d'électrodes) de façon schématique implantée dans le coeur d'un patient.

Sur la figure, la référence 10 désigne le coeur, avec sa cavité auriculaire 20 et sa cavité ventriculaire 30.

Le dispositif implanté comprend un générateur d'impulsions 60 et un système d'électrodes.

Ce système d'électrodes comprend :
- une électrode auriculaire 40 placée au voisinage de l'oreillette 20 ou dans celle-ci, et reliée au générateur 60 par un conducteur 45 connecté à une entrée/sortie 46 du générateur,
- une électrode ventriculaire 50 placée dans le ventricule droit 30 et reliée au générateur 60 par un conducteur 55 connecté à une entrée/sortie 56 du générateur,
- de façon optionnelle, une électrode "patch" 70 reliée par un conducteur 75 à une sortie 76 du générateur,
- de façon optionnelle, le boîtier 80 du générateur 60 peut être un boîtier métallique formant électrode ; plus précisément, l'une au moins des électrodes 70 et 80 peut être utilisée pour assurer le retour du courant lors de l'application du choc, les deux électrodes 70 et 80 pouvant être si on le souhaite utilisées concurremment.

Tous ces éléments et leurs rôles sont connus en eux-mêmes dans l'art antérieur et ne nécessitent pas de plus ample description.

Le dispositif peut envoyer au coeur l'impulsion de haute énergie (cardioversion ou défibrillation) selon diverses configurations d'électrodes par des commutations appropriées qui sont réalisées par les circuits logiques et analogiques internes du générateur d'impulsions 60. Le FR-A-2 711 064 décrit la manière dont ces différentes commutations peuvent être, d'une part, choisies pour optimiser l'effet du choc et, d'autre part, réalisées électriquement, par exemple au moyen de composants à l'état solide tels que MOSFETs, IGBTs, SCRs ou composants analogues.

Les diverses configurations possibles, déjà évoquées plus haut, sont les suivantes :
a) l'impulsion est délivrée entre les électrodes 40 et 50,
b) l'impulsion est délivrée entre l'électrode 50 et l'électrode 70 ou l'électrode 80 ; en variante, elle peut être délivrée entre, d'une part, l'électrode 50 et, d'autre part, les électrodes 70 et 80 réunies ensemble, cette commutation s'effectuant sous l'action de la logique interne du générateur 60,
c) l'impulsion est délivrée entre, d'une part, l'électrode 50 et, d'autre part, les électrodes 40 et 80 réunies ensemble ; dans une variante, l'électrode 70 peut être également réunie à l'ensemble formé par les électrodes 40 et 80 ;
d) l'impulsion est délivrée entre, d'une part, les électrodes 40 et 50 réunies ensemble et, d'autre part, l'électrode 70 ou l'électrode 80 ; en variante, les électrodes 70 et 80 peuvent être également reliées entre elles.

Toutes les connexions d'électrodes sont décidées et exécutées sous l'action de la logique interne du générateur 60.

L'invention s'applique au cas de figure dans lequel un choc de cardioversion doit être appliqué.

Dans ce cas, dans un premier temps, la logique interne du générateur 60 déconnecte l'entrée/sortie 46, si bien que l'électrode auriculaire 40 va se trouver à un potentiel flottant par rapport au reste du dispositif.

Dans un second temps, la logique interne examine quelle aurait été la configuration d'électrodes normalement choisie, c'est-à-dire la configuration la mieux appropriée à la situation du patient selon les critères habituels.

Si, dans la configuration qui aurait été normalement choisie, l'électrode 40 aurait dû servir d'électrode de retour du courant, alors on modifie la configuration d'électrode pour substituer à l'électrode auriculaire 40 l'électrode 80 et/ou l'électrode 70 comme électrode de retour.

Si, en revanche, la configuration normalement choisie n'implique pas l'utilisation de l'électrode 40 comme électrode de retour, alors on ne modifie rien à la configuration, qui reste celle initialement programmée.

Bien entendu, lorsque l'on doit délivrer un choc de défibrillation, c'est-à-dire un choc de plus haute énergie, l'électrode auriculaire 40 est automatiquement reconnectée au dispositif afin de proposer l'ensemble des configurations a), b), c) et d), la technique particulière de l'invention ne s'appliquant que dans le cas de la délivrance d'un choc de cardioversion.

La mise en oeuvre de la déconnexion de l'entrée/sortie 46 peut être opérée par un dispositif du même type que celui du FR-A-2 711 064 précité, la seule modification à apporter concernant la logique de commande, dont la programmation doit être adaptée de manière à réaliser les actions recherchées. Cette modification de la programmation est à la portée de l'homme du métier et ne sera pas décrite plus en détail.

## Revendications

1. Défibrillateur/cardioverteur implantable, comprenant :
- une électrode auriculaire (40) adaptée à être placée au voisinage ou à l'intérieur de l'oreillette (20),
- une électrode ventriculaire (50) adaptée à être placée à l'intérieur du ventricule (30),
- au moins une électrode adaptée à être placée externe au coeur (70, 80), et
- un boîtier générateur d'impulsions (60), comportant :
. des moyens de délivrance d'une énergie de défibrillation ou de cardioversion, et
. des moyens de commutation de configuration d'électrodes, qui déterminent entre quelles électrodes doit être appliquée l'énergie de défibrillation ou de cardioversion,
**caractérisé en ce que** les moyens de commutation comprennent en outre des moyens pour, lorsqu'une énergie de cardioversion doit être délivrée :
- déconnecter l'électrode auriculaire des moyens de délivrance de l'énergie de défibrillation ou de cardioversion si bien que l'électrode auriculaire se trouve à un potentiel flottant, et
- si la configuration d'électrodes déterminée par les moyens de commutation prévoit la commutation de l'électrode auriculaire, substituer à cette électrode l'une au moins des électrodes externes au coeur.

## Patentansprüche

1. Implantierbarer Defibrillator/Kardioverter, aufweisend:
- eine aurikuläre Elektrode (40), die geeignet ist, um in der Nähe oder im Inneren des Vorhofs (20) angeordnet zu werden,
- eine ventrikuläre Elektrode (50), die geeignet ist, im Inneren des Ventrikels (30) platziert zu werden,
- zumindest eine Elektrode, die geeignet ist, um außerhalb des Herzens (70, 80) angeordnet zu sein, und
- ein Pulsgeneratorgehäuse (60), aufweisend:
- Mittel zum Liefern einer Defibrillationsenergie oder Kardioversionsenergie, und
- Kommutierungsmittel einer Konfiguration von Elektroden, die bestimmen, zwischen welchen Elektroden die Defibrillationsenergie oder Kardioversionsenergie angelegt werden muss,
**dadurch gekennzeichnet, dass** die Kommutierungsmittel unter anderem Mittel aufweisen, um, wenn eine Kardioversionsenergie zugeführt werden muss:
- die aurikuläre Elektrode von Mitteln zum Liefern einer Defibrillationsenergie oder Kardioversionsenergie zu unterbrechen, so dass sich die aurikuläre Elektrode bei einem schwankenden Potential befindet, und
- wenn die Konfiguration von durch die Kommutierungsmittel bestimmten Elektroden die Kommutierung der aurikulären Elektrode vorsieht, mit dieser Elektrode zumindest eine der Elektroden außerhalb des Herzens zu substituieren.

## Claims

1. Implantable defibrillator/cardioverter, comprising:
- an atrial electrode (40) adapted to be placed in the vicinity of or inside the atrium (20);
- a ventricular electrode (50) adapted to be placed inside the ventricle (30);
- at least one electrode adapted to be placed external to the heart (70, 80); and
- a pulse generator casing (60), comprising:
· means for delivering a defibrillation or cardioversion energy, and
· means for switching between electrode arrangements, that determine between which electrodes the defibrillation or cardioversion energy must be applied,
**characterized in that** the switching means further comprises means for, when a cardioversion energy must be applied:
- disconnecting the atrial electrode from the means for delivering a defibrillation or cardioversion energy so that the atrial electrode is allowed to be under a floating potential, and
- if the electrode arrangement determined by the switching means provides for the commutation of the atrial electrode, replace this electrode by at least one of the electrodes external to the hearth.
